# EUROPÄISCHE PATENTSCHRIFT

(11) **EP 0 839 796 B1**
(45) Veröffentlichungstag und Bekanntmachung des Hinweises auf die Patenterteilung: **12.06.2002**
(21) Anmeldenummer: 97118323.1
(22) Anmeldetag: 22.10.1997
(51) Int. Cl.: C07C 209/16, C07C 211/18

(54) **Verfahren zur Herstellung von 1.4 Bis(aminomethyl) cyclohexan**
Process for the preparation of 1,4-bis(aminomethyl) cyclohexane
Procédé pour la préparation de 1,4-bis(aminométhyl) cyclohexane

(30) Priorität: 04.11.1996 DE 19645360; 29.11.1996 DE 19649658
(43) Veröffentlichungstag der Anmeldung: 06.05.1998
(73) Patentinhaber: BAYER AG, 51368 Leverkusen (DE)
(72) Erfinder: Waldmann, Helmut Dr., 52385 Nideggen (DE); Dahmer, Jürgen Dr., 47800 Krefeld (DE); Nachtkamp, Klaus Dr., 40593 Düsseldorf (DE); Bazanov, Anatoly Prof., 193318 St. Petersburg (RU); Timofeev, Alexandre Dr., 195253 St. Petersburg (RU); Zubritskaya, Natalja Dr., 190068 St. Petersburg (RU); Terechtchenko, Gennady Prof., 195220 St. Petersburg (RU)

(56) Entgegenhaltungen:
- US-A- 3 143 570

## Beschreibung

Die Erfindung betrifft ein Verfahren zur Herstellung von 1,4-Bis(aminomethyl)-cyclohexan.

Es ist bekannt 1,4-Bis(aminomethyl)cyclohexan in einem Zweistufenverfahren aus Dicyanobenzol herzustellen (z.B. DE-A 30 03 730). Im ersten Schritt wird Dicyanobenzol katalytisch zu 1,4-Bis(aminomethyl)benzol hydriert, welches anschließend katalytisch zu 1,4-Bis(aminomethyl)cyclohexan hydriert wird. Nachteilig ist u.a., daß das Ausgangsmaterial mit Cyanwasserstoff hergestellt werden muß.

Ein erster Vorschlag zu einer Synthese ohne Cyanide geht von 1,4-Bis-hydroxymethylcyclohexan aus (US-P 3 143 570), erweist sich aber als technisch unbefriedigendes Verfahren.

Es wurde nun ein einfacher Weg gefunden, 1,4-Bis(aminomethyl)cyclohexan herzustellen.

Gegenstand der Erfindung ist ein Verfahren zur Herstellung von 1,4-Bis-(aminomethyl)cyclohexan, das dadurch gekennzeichnet ist, daß eine 40 bis 80 gew.-%ige wäßrige Lösung von 1,4-Bis(hydroxymethyl)cyclohexan in Gegenwart eines Nickel/Kupfer/Chrom-Katalysators unter einem Druck von 100 bis 250 bar bei einer Temperatur von 150 bis 250°C mit Wasserstoff/Ammoniak umgesetzt wird.

Der feinteilige Katalysator besteht aus 60 bis 50 mol-% Nickel, 14 bis 17 mol-% Kupfer und 26 bis 33 mol-% Chrom.

Die Umsetzung wird bei einer Temperatur von 150 bis 250°C und einem Druck von 150 bis 250 bar durchgeführt, gegebenenfalls unter Inertgas (z B N₂).

### Beispiele

### Beispiel 1

Das Verfahren wird in einem Durchflußreaktor mit Festbettkatalysator durchgeführt. Der Reaktor wurde mit 100 cm³ Volumen eines Katalysators bestehend aus 57 mol-% Nickel, 14 mol-% Kupfer und 29 mol-% Chrom beschickt und mit einem Wasserstoffstrom reduziert. Die Reaktanten wurden mit einer stündlichen Rate von 100 l Wasserstoff, 100 cm³ flüssigen Ammoniak und 20 cm³ einer 50 %igen wäßrigen Lösung von 1,4-Bis(hydroxymethyl)-cyclohexan zugegeben.

Die Temperatur wurde bei 190°C bei einem Druck von 200 bar gehalten während der Durchführung der Reaktion. Nach Entfernung des Wassers und Ammoniak aus der Mischung wurden pro Stunde 9,9 g eines Produktes erhalten, das 60 Gew.-% 1,4-Bis(aminomethyl)cyclohexan und 20,3 Gew.-% 1,4-Bis(hydroxymethyl)cyclohexan enthielt. Die Umsatzrate betrug 80,1 % und die Selektivität bezüglich der Bildung von 1,4-Bis(aminomethyl)cyclohexan war 74,5 mol-%.

### Beispiel 2

Verfahrensdurchführung und Katalysator waren die gleichen wie in Beispiel 1. Die Reaktanden wurden mit einer stündlichen Flußrate von 100 1 Wasserstoff, 100 cm³ flüssigen Ammoniak und 13 cm³ einer 70 %igen wäßrigen Lösung von 1,4-Bis(hydroxymethyl)cyclohexan zugegeben. Die Temperatur betrug 190°C und der Druck 200 bart. Nach Emfernen des Wassers und Ammoniaks wurden pro Stunde eine Mischung von 8,8 g eines Produktes erhalten, welches 71,4 Gew.-% 1,4-Bis(aminomethyl)cyclohexan und 11,2 Gew.-% 1,4-Bis(hydroxymethyl)cyclohexan enthielt. Die Umsatzrate betrug 89,2 % und die Selektivität bezüglich 1,4-Bis-(aminomethyl)cyclohexan betrug 78,3 mol-%.

### Beispiel 3

Das Verfahren wurde wie in Beispiel 1 durchgeführt. Der Reaktor wurde mit 100 cm³ Volumen eines Katalysatorpulvers bestehend aus 50 mol-% Nickel, 17 mol-% Kupfer und 33 mol-% Chrom beschickt. Die Reaktanden wurden mit einer stündlichen Zuflußrate von 100 l Wasserstoff, 100 cm³ flüssigen Ammoniak und 13 cm³ einer 50 gew.-%igen wäßrigen Lösung von 1,4-Bis(hydroxymethyl)-cyclohexan beschickt. Die Temperatur betrug 190°C und der Druck 200 bar während der Dauer des Verfahrens. Nach Entfernen des Wassers und Ammoniak wurden mit einer stündlichen Rate von 6,4 g ein Produkt erhalten, welches 53 Gew.-% 1,4-Bis(aminomethyl)cyclohexan und 17,8 Gew-% 1,4-Bis(hydroxymethyl)cyclohexan enthielt. 1,4-Bis(hydroxymethyl)cyclohexan wurde zu 82,6 % umgesetzt und die Selektivität bezüglich der Bildung von 1,4-Bis(aminomethyl)-cyclohexan betrug 63,5 mol-%.

## Patentansprüche

1. Verfahren zur Herstellung von 1,4-Bis(aminomethyl)cyclohexan, **dadurch gekennzeichnet, daß** eine 40 bis 80 gew-%ige wäßrige Lösung von 1,4-Bis(hydroxymethyl)cyclohexan in Gegenwart eines feinteiligen Nickel/Kupfer/Chrom-Katalysators unter einem Druck von 100 bis 250 bar bei einer Temperatur von 150 bis 250°C mit Wasserstoff/Ammoniak umgesetzt wird.

## Claims

1. Process for the production of 1,4-bis(aminomethyl)cyclohexane, **characterised in that** a 40 to 80 wt.% aqueous solution of 1,4-bis(hydroxymethyl)cyclohexane is reacted with hydrogen/ammonia in the presence of a finely divided nickel/copper/chromium catalyst under a pressure of 100 to 250 bar at a temperature of 150 to 250°C.

## Revendications

1. Procédé pour la préparation du 1,4-bis(aminométhyl)-cyclohexane, **caractérisé en ce que** l'on fait réagir une solution aqueuse de 1,4-bis(hydroxyméthyl)cyclohexane à une concentration de 40 à 80 % en poids avec le couple hydrogène/ammoniac sous une pression de 100 à 250 bar et à une température de 150 à 250°C en présence d'un catalyseur au nickel/cuivre/chrome en fines particules.
